Europäisches Patentamt

European Patent Office

Office européen des brevets

(11) Publication number: **0 377 722 B1**

(19)

# EUROPEAN PATENT SPECIFICATION

(12)

(45) Date of publication of patent specification: **15.12.93**  (51) Int. Cl.<sup>5</sup>: **A61K 39/118**, A61K 45/06, G01N 33/569

(21) Application number: **89907760.6**

(22) Date of filing: **27.06.89**

(86) International application number:
**PCT/FI89/00122**

(87) International publication number:
**WO 90/00061 (11.01.90 90/02)**

(54) **METHOD FOR THE TREATMENT AND THE DIAGNOSIS OF CORONARY HEART DISEASE.**

(30) Priority: **27.06.88 FI 883067**

(43) Date of publication of application:
**18.07.90 Bulletin 90/29**

(45) Publication of the grant of the patent:
**15.12.93 Bulletin 93/50**

(84) Designated Contracting States:
**AT BE CH DE FR GB IT LI LU NL SE**

(56) References cited:
**EP-A- 0 059 624**
**EP-A- 0 192 033**

**NATL. LIBRARY OF MEDICIN NLM, Database Medline; P. RIBEIRO et al., acc. no. 86081839&NUM;**

**NATL. LIBRARY OF MEDICIN NLM, Database Medline; A ISAKOV et al., aCc. no. 83006426&NUM;**

(73) Proprietor: **LABSYSTEMS OY**
**Pulttitie 8**
**SF-00881 Helsinki(FI)**

(72) Inventor: **SAIKKU, Pekka**
**Vihdintie 13 A 3**
**SF-00300 Helsinki(FI)**
Inventor: **LEINONEN, Maija**
**Vihdintie 13 A 3**
**SF-00300 Helsinki(FI)**

(74) Representative: **Bunke, Holger, Dr.rer.nat.**
**Dipl.-Chem. et al**
**Prinz & Partner**
**Manzingerweg 7**
**D-81241 München (DE)**

NATL. LIBRARY OF MEDICIN NLM, Database Medline; J.H. SVENDSEN et al., acc. no. 88107286&NUM;

NATL. LIBRARY OF MEDICIN NLM, Database Medline; L.J. WALKER, acc. no. 87100698&NUM;

NATL. LIBRARY OF MEDICIN NLM, Database Medline; C.C. KUO, acc. no. 88208346&NUM;

**Description**

This invention relates to a method for screening, diagnosis and monitoring of coronary heart disease and myocardial infarction as well as to the use of drugs effective against chlamydia.

In heart infarction a clog in coronary artery leads to ischemia and partial gangrene of the heart muscle. In angina pectoris the patient suffers from chronich tightness (atherosclerosis) of coronary arteries which lead to ischemia and to typical pain symptoms. There are several factors which are generally known to increase the risk of getting angina pectoris or heart infarction. There are e.g. high blood pressure, smoking, and low HDL cholesterol content and high LDL cholesterol content of blood.

Nowadays the risks of heart infarctions are detected e.g. by measuring total cholesterol and HDL and LDL cholesterol in blood.

The patients with heart infarctions are nowadays treated e.g. by thrombolytic agents, by vasoactive drugs like $\beta$-blocking agents, cathecol amines, nitroglycerines, or cardioactive drugs like digitalis. A special risk for the patient is a renewed infarction within a few days from the first infarction. Nowadays this is tried to get prevented primarily by anticoagulants and drugs mentioned above.

The prophylaxis of heart infarctions nowadays comprises mainly alteration of dietary and smoking habits, by $\beta$-blocking agents and drugs affecting cholesterol metabolism.

It has also been suggested that circulating or tissue-deposited immune complexes might play pathogenic role in the development of chronic coronary heart disease and acute myocardial infarction (Füst G, Szondy E, Sekely J, Nanai I, Gerö S, Studies on the occurrence of circulating immune complexes in vascular diseases, Atherosclerosis 1978:28:181-190; Farrel C, Bloth B, Nielsen H, Dougharty H, Lundman T, Svehag S-E, A survey for circulating immune complexes in patients with acute myocardial infarction, Scand J Immunol 1977:6:1233-1240; Cristea A, Rees H, Niculescu F, Bedeleanu D, Vlaicu R, Characterization of circulating immune complexes in heart disease, Immunol Lett, 1986:13:45-49). Immune complexes are thought to act by the activation of complement and thus releasing chemotactic and anaphylactic cleavage products leading to inflammation, platelet aggregation and vasospasm. The presence of immune complexes has been demonstrated in 50 to 60 % of patients after myocardial infarction. However, thus far the antigen in immune complexes has not been characterized except those possibly associated with tissue autoantigens after the injury.

Heart infarction has long been one of the major causes of death. Acute infarction can attack unexpectedly also on relatively young people, which makes the disease especially harmful. Thus there has already long been urgent need of means for screening possible victims of heart infarctions, and for treatment and prophylaxis of coronary heart disease.

Therefore, the problem underlying the invention is to provide a method for screening, diagnosis and monitoring of coronary heart disease or myocardial infarction.

According to the invention, this problem is solved by a method for screening the risks of heart infarction, for the diagnosis of myocardial infarction and coronary heart disease, and for the monitoring of the treatment of patients with acute myocardial infarction which is characterized in that a sample of a patient is tested for the presence of chlamydia or chlamydial antibodies.

It has been found that chlamydial infections play a significant role in the pathogenesis of coronary heart disease. Especially significant are Chlamydia TWAR infections.

Heart infarctions can thus often be prevented by treating possible chlamydial infections. This can be done especially by administrating antibiotics which are effective against chlamydia organisms. Chlamydial infections, on the other hand, can be principally prevented by vaccination. For the prevention of heart infarction risks systemic immunity is probably sufficient.

Therefore, it is a further object of the invention that drugs effective against chlamydia are used for the manufacture of a pharmaceutical preparation to be used in the treatment or prophylaxis of coronary heart disease or myocardial infarction.

Detection of the possible chlamydial infection can also be used in the therapy of patients who have just undergone a heart infarction. If the possible chlamydial infection is treated, the risk of getting a new infarction during the recovery can be remarkably decreased. Such a secondary infarction is often highly dangerous.

Chlamydia are obligatory intracellular gram-negative bacteria. They have been so far divided into two species: C. trachomatis and all the others lumped into a single species of C. psittaci. The last mentioned heterogeneous group also contains so called TWAR strains (Grayston JT, Kuo CC, Wang SP, Altman J, A new Chlamydia psittaci strain, TWAR, isolated in acute respiratory tract infections, N Engl J Med 315:161, 1986). However, quite recently it has been indicated that TWAR strains possess so unique structural and biological characteristics that a status of a completely new chlamydial species has been proposed to them (Kuo CC, Chen HH, Wang SP, Grayston JT, Identification of a new group of Chlamydia psittaci strains called TWAR, J Clin Microbiol 24:1034, 1986; Campbell LA, Kuo CC, Grayston JT, Characterization of the new Chlamydia agent, TWAR, as a unique organism by restriction endonuclease analysis and DNA-DNA hybridization, J Clin Microbiol 25:1911, 1987; Chi EY, Kuo CC, Grayston JT, Unique ultrastructure in the elementary body of Chlamydia sp. strain TWAR, J Bacter 169:3757, 1987; Cox R, Kuo CC, Grayston JT, Campbell LA, Deoxyribonucleic acid relatedness of Chlamydia specis strain TWAR to Chlamydia trachomatis and Chlamydia psittaci, Int J System Bact 38:265, 1988). Chlamycia pneumoniae is the suggested name (Grayston JT, Kuo CC, Campbell LA, Wang sp, Chlamydia pneumoniae sp. nov. for Chlamydia sp. strain TWAR, Int J System Bact 1989:39:88-90).

Studies have revealed the unique nature of TWAR chlamydia. It is difficult to grow, HeLa 229 being the best cell line known so far. Samples should be frozen stepwise for storage at -70 °C. Cultivation demands DEAE-dextran treatment, high centrifugal forces, and incubation at 35 °C in the presence of cycloheximide. The inclusions are stained on the 3rd day with fluorescent monoclonal antibody. In preliminary screening, group-specific clone can be used, but final proof of an isolate beeing a TWAR strain demands use of species-specific monoclone. Adaptation of isolates to cell cultures can be a prolonged process and often demands blind passages in yolk sac, where TWAR strains grow relatively readily, but usually without killing of embryos. Isolates obtained so far have behaved identically in characterization tests despite the differences in time of original isolation or in geographical origin. They possess the common chlamydial lipopolysaccharide (LPS) antigen and are resistant to sulfa drugs, but can easily be separated from C. psittaci by serological methods, and nucleic acid restriction enzyme and hybridization patterns. No plasmid has been found. In electron microscopy they show the typical elementary body (EB) - reticulate body (RB) cycle inside a chlamydial inclusion, but the EB of TWAR chlamydia demonstrates a unique pearshaped structure with a loose outer membrane and a large periplasmic space often containing miniature bodies.

The final criteria justifying the proposal of a new species is supported by the absence of marked DNA homology with the two chlamydial species described earlier.

All chlamydia cause several infectious diseases both in man and animals. C. trachomatis is the causative agent of trachoma in developing countries and is most common cause of genital infections in developed countries. C. psittaci, besides being an important veterinary pathogen, causes respiratory infections in man. TWAR chlamydia has been found out to be an ubiquitous pathogen of humans, and has so far been associated with respiratory tract infections, especially pneumonias (Saikku P, Wang SP, Kleemola M, Brander E, Rusanen E, An epidemic of mild pneumonia due to an unusual strain of Chlamydia psittaci, J Infec Dis 151:832, 1985; Kleemola M, Saikku P, Visakorpi R, Wang SP, Grayston JT, Epidemics of pneumonia caused by TWAR, a new Chlamydia organism, in military trainess in Finland, J Infect Dis 157:230, 1988; Grayston JT; Wang SP, Kuo CC, Altman J, Marrie T, Mordhorst C, Clinical findings in TWAR respiratory tract infections, In: D Oriel et al, eds., Chlamydial Infections, p 337, Cambridge University Press, Cambridge 1986; Marrie TJ, Grayston JT, Wang SP, Kuo CC, Pneumonia associated with the TWAR strain of Chlamydia, Ann Int Med 106:507, 1987; Ekman MR, Saikku P, Kleemola M, Visakorpi R, Kuo CC, Grayston JT, Mild and asymptomatic respiratory infections with Chlamydia TWAR during a military pneumonia epidemic, In: Chlamydia Research I, Proc 1st Symp European Soc for Chlamydia Res, p. 56, Almqvist & Wiksell, Stockholm 1988).

Chlamydia TWAR infections are difficult to diagnose. The isolation of organisms by cell culture is not easy to perform. At present the most reliable method for the etiological diagnosis of TWAR infections is microimmunofluorescence method for TWAR antibodies (Wang SP, Grayston JT, Microimmunofluorescence serological studies with the TWAR organism, In "Chlamydial Infections", ed. D. Oriel et al. 1986;329-32, Cambridge Univ Press).

By using micro-IF, the role of TWAR strains as respiratory pathogen has been documented. The complement fixation test (CF) and enzymeimmunoassay utilizing Re-LPS, shown to be cross-reactive with chlamydial LPS, can be used for serological diagnosis of TWAR infections. However, these two methods pick up all chlamydial infections, because they measure antibodies to LPS common to all chlamydial species.

Chlamydia have been recognized as cardiovascular pathogens and implicated also in various valvular heart diseases. There are reports on myocarditis associated with both C. trachomatis (Grayston JT, Mordhorst CH, Wang SP, Childhood myocarditis associated with Chlamydia trachomatis infection, JAMA 1981;246:2823-27; Ringel RE, Brenner JT, Rennels MB, Huang SW, Wang SP, Grayston JT, Berman MA, Serologic evidence for Chlamydia trachomatis myocarditis, Pediatrics 1982;70:54-56) and C. psittaci infections (Dymock IW, Lawson JM, MacLennan WJ, Ross CAC, Myocarditis associated with psittacosis, Brit J Clin Pract 1971;25:240-42; Thomas DJB, MacDonald PJ, Fowler JM, Mistaken diagnosis - psittacosis myocarditis, Practioneer 1977;218:394-98).

4

Endocarditis has been demonstrated in connection of infections caused by both species (Editorial, Chlamydial endocarditis, Lancet 1980;i:132; van der Bel-Kahn JM, Watanakunakorn C, Menefee MG, Long HD, Dieter R. Chlamydia trachomatis endocarditis, Amer Heart J 1978;95:627-36) and major arterial emboli caused by C. psittaci have also been described (Bromage D, Jeffries DJ, Philip G, Embolic phenomena in chlamydial infection, J Infection 1980;2:151-59). In addition, even epidemiological association of primary myocardial disease and widespread chlamydial infection has been indicated already in 1967 (Sutton GC, Morrissey RA, Tobin JR, Anderson TO, Pericardial and myocardial disease associated with Serological evidence of infection by agents of the psittacosis-lymphogranuloma venerum group (Chlamydiacae), Circulation 1967;36:830-38; Sutton GC, Demakis JA, Anderson TO, Morrissey RA, Serological evidence of a sporadic outbreak in Illinois of infection by Chlamydia (psittacosis-LGV agent) in patients with primary myocardial disease and respiratory disease, Amer Heart J 1971;81:597-607).

Now it has been found that chlamydia, especially Chlamydia TWAR, are associated also with coronary heart disease and especially with acute infarctions.

The suggested pathogenetic mechanism of Chlamydia TWAR in chronic heart disease and infarction is as follows:

After respiratory infection caused by TWAR, some people develop chronic TWAR infection in which bacteria reside in the cells of reticuloendothelial system either in lungs or heart vascular system. Chlamydia shed their components like (LPS) that are released from infected cells. Antibodies to chlamydial LPS present in blood are bound to circulating LPS forming immunocomplexes and these complexes further increase inflammatory response on vascular endothelium leading to coronary heart disease. In the acute phase of myocardial infarction, antibody levels to LPS in serum are very low suggesting the presence of immunocomplexes and after infarction profound antibody response to LPS can be demonstrated suggesting either liberation of tissue-deposited immunocomplexes or liberation of antibodies from immunocomplexes or actual response to LPS.

It is further believed that there may be also other still unknown types of chlamydia involved.

Thus by the treatment of chronic chlamydial infection by drugs effective against chlamydia chronic coronary heart disease could be cured. On the other hand by the treatment with pharmaceuticals effective against inflammatory effects of chlamydial components myocardial infarction could be prevented.

Suitable drugs against chlamydia are e.g. tetracyclines, erythromycin, rifampicillin, and fluoroquinolenes. It is not known at present what is the best treatment for chronic chlamydial infections. Recurrencies or reactivation of infections are frequently seen after proper treatment with tetracyclin or erythromycin. Evidently longer treatment (up to four weeks) and higher doses of antibiotics or combination of different antibiotics is needed for eliminating chronic chlamydial infections.

Risks of heart infarcts in a patient can also be screened in accordance with the invention. This method comprises taking a sample of blood, sputum, pleural fluid, urine, sample by bronchoscopy or bronchoalveolar lavation or biopsy sample from heart, vascular endothelium, spleen, liver or lungs and testing the sample for the presence of chlamydia, chlamydial components like LPS or membrane proteins or substances produced by chlamydia like exopolysaccharide or substances produced by host cells by induction of chlamydia or chlamydial antibodies. Suitable techniques are culture isolation, immunochemical methods like solid phase or liquid immunoassays like enzymeimmunoassay, radioimmunoassay or fluoroimmunoassay, immunofluorescence methods, histological and immunohistochemical staining methods, electronmicroscopy, immunoelectronmicroscopy, or nucleic acid hybridization. This method can also be used for the diagnosis of myocardial infarction and coronary heart disease, as well as for the monitoring of treatment of patients with acute myocardial infarction or coronary heart disease.

The disease can be e.g. cerebral infarction or vascular embolization.

According to one aspect of the invention vaccine can be prepared and used in the prophylaxis.

Association of Chlamydia TWAR with chronic coronary heart disease and myocardial infarction

Patients and controls

Patients were divided to two groups. Acute myocardial infarction (AMI) group consisted of 40 consecutive young and middle aged men admitted to the Helsinki University Central Hospital because of an acute myocardial infarction. The inclusion criteria were: male gender (age 50 years or less), resident in Helsinki or immediate neighbourhood, onset of symptoms within 48 hours prior to the addmittance, and verification of acute myocardial infarction by typical changes in electrocardiogram (ECG) and elevation of serum creatinine phosphokinase MB-isoenzyme activity. The mean age of the patients was 44.5 years (range 34 - 50 years).

Chronic coronary heart disease (CCHD) group consisted of 30 consecutive male patients addmitted to the hospital for coronay angiography because of severe chronic symptoms of angina pectoris. Subjects with acute myocardial infarction within 6 months before entry to the study were excluded. All patients were living in the same area as AMI group and all had chronic coronary heart disease documented both clinically and angiographically. The mean age was 41 years (range 28 to 50 years).

Controls were 41 healthy men recruited as a computer based random sample from the official inhabitant records of the city of Helsinki from the same area as patients. Sixty per cent of those invited agreed to participate. The most common cause for refusal was lack of time. None of the controls had any signs or symptoms of coronary heart disease as judged by negative history and a normal resting ECG. Except for a man who was treated with a beta-blocking agent for hypertension, all the subjects were free of chronic disease or treatments. The controls were selected to represent the same age range (30 to 50, mean age 39.1 years) as the patients, and they were invited to the examination sequentially one at a time within 2 to 4 weeks after the adimission of index case with acute myocardial disease.

Serum samples for determination of antibodies were taken on admission and 4 weeks later from all individuals except for two AMI patients (one Succumbed and one dropped out). In addition, 3rd serum sample taken 3 months after admission was obtained from AMI patients.

Micro-immunofluorescence (micro-IF) test

A simplified modification of micro-IF assay of WANG & GRAYSTON utilising only TWAR antigen (AR-39, obtained from Washington Research Foundation, Seattle, WA, U.S.A.) was used for determination of chlamydia TWAR antibodies (Wang SP, Grayston JT, Immunologic relationship between genital TRIC, lymphogranuloma venerum, and related organism in a new microtiter indirect immunofluorescence method, Am J Ophthalmol 1970;70:367-74). Fluorescein-isothiocyanate labelled conjugates against human immunoglobulins (λ-chain-specific, Kallestad, Austin, U.S.A.; μ-chain-specific, Dakopatt, Denmark; α-chain-specific, Orion Diagnostics, Finland) were used with amidoschwartz as counterstain. The sera were titrated in two-fold dilutions starting from 1:8, and the limit titer of positivity was considered as 1:32 (Wang SP, Grayston JT, Microimmunofluorescence serological studies with the TWAR organism. In "Chlamydial Infections", ed. D. Oriel et al. 1986;329-32, Cambridge Univ Press).

Enzyme-immunoassay for determination of antibodies against chlamydial lipopolysaccharide (LPS-EIA)

Details and validation of the method have been described earlier (Puolakkainen M, Saikku P, Leinonen M, Nurminen M, Mäkelä PH, Chlamydial pneumonitis and its serodiagnosis in infants, J Infec Dis 1984:149:598-604). Briefly, the antigen was LPS from core-deficient mutant of enterobacteria (Re-LPS from Salmonella minnesota, Calbiochem, U.S.A.) shown to crossreact with chlamydial group antigen (Nurminen M, Leinonen M, Saikku P, Mäkelä PH, The genusspecific antigen of Chlamydiae: Resemblance to the lipopolysaccharide of enteric bacteria, Science 1983:220:1279-81). Alkaline phosphatase labelled conjugates against human IgG, IgM and IgA were obtained from Orion Diagnostics (Espoo, Finland). Titers were expressed as highest dilutions giving OD-value of 0.3.

Statistical-analysis

Sample proportions were compared by chi-square test with Yates' correction. When the minimum estimated expected value was under five, Fisher's exact test was used.

TWAR IF-antibodies

Both patient groups had significantly more IgG and IgA antibodies than the control group (table 1). The prevalence of IgG antibodies in titers of ≥ 1:32 was in AMI group 35/40 (p < 0.05) and in CCHD 26/30 (p < 0.01) when compared to 24/41 of controls. The respective geometric mean titers (GMT) were 72.5, 76.4 and 24.5. The patient groups had also significantly more elevated IgG titers (≥ 128) againts the TWAR agent than the controls (20/40 and 14/30 vs. 6/41, p < 0.0007). The prevalences of IgA were 11/40 in AMI group and 10/30 in CCHD group vs. 3/41 in controls (p < 0.0004), and the GMTs were 15.7, 15.2 and 6.2, respectively. When both markers, elevated IgG and presence of IgA were combined, the significance level was p < 0.0001 (27/40 and 15/30 vs. 7/41 in controls). IgM antibodies were not found.

6

EIA antibodies

The majority of patients did not have IgG or IgA antibodies against Re-LPS, and their IgM antibody levels were low when compared to the controls. 26/39 patients with AMI showed a marked IgM antibody response to Re-LPS between first and second serum samples obtained four weeks apart. This response was also seen in IgG and IgA antibodies in somewhat lesser extent. Antibody titers declined in third serum sample. None of the CCHD patients and only one control showed seroconversion against Re-LPS in their paired serum samples (table 1).

Table 1. The presence of elevated IgG or IgA titers against chlamydia TWAR and significant ($\geq$ 3-fold) seroconversions between paired sera in enzyme immunoassay (EIA) test with Re-LPS antigen in the patient and control groups. AMI = acute myocardial infarction, CCHD = chronic coronary heart disease.

| | AMI (No.=40) | CCHD (No.=30) | Control (No.=41) | Significance AMI vs. CCHD | AMI vs. control |
|---|---|---|---|---|---|
| TWAR IgG $\geq$ 32 | 34 | 26 | 25 | ns* | 0.02 |
| TWAR IgG $\geq$ 128 | 20 | 14 | 6 | ns | 0.0007 |
| TWAR IgG GMT** | 78.79 | 78.79 | 30.42 | ns | 0.0002 |
| TWAR IgA $\geq$ 32 | 18 | 11 | 4 | ns | 0.0004 |
| TWAR IgA GMT | 20.39 | 17.96 | 10.67 | ns | 0.0008 |
| TWAR IgG/A $\geq$ 128/$\geq$ 32 | 27 | 15 | 7 | ns | 0.00001 |
| Re-LPS EIA $\geq$ 3-fold increase | 26/39 | 0/30 | 1/41 | 0.00001 | 0.00001 |

* = not significant (p > 0.05)

** = geometric mean titer

Microimmunofluorescence test with TWAR elementary bodies as antigen is a reliable method for measuring specific antibodies to this serologically unique new chlamydial species (Wang SP, Grayston JT. In "Chlamydial Infections", ed. D. Oriel et al. 1986;329-32, Cambridge Univ Press; Kuo CC, Chen HH, Wang SP, Grayston JT, Identification of a new group of Chlamydia psittaci strains called TWAR, J Clin Microbiol 1986;24:1034-37). To avoid contamination with tissue lipids present in chlamydial group antigen preparations produced from yolk sacs or tissue cultures, Re-LPS antigen known to share one carbonhydrate epitope with chlamydial LPS were used in EIA (Nurminen M, Leinonen M, Saikku P, Mäkelä PH, Science 1983;220:1279-81; Editorial, Psittacosis of non-avian origin, Lancet 1984;ii:442-43). It has also been found that antibody responses to Re-LPS antigen are not found in enterobacterial infections like septicemia and

7

pyelonephritis. Positive antibody responses to Re-LPS have been confirmed to be of chlamydial origin by micro-IF. Thus it is considered that the antibody findings with these different types of test used are diagnostic for chlamydial infection.

Chlamydia TWAR is known to occur in Finland as widespread epidemics, which have conveniently monitored in military settings. Due to these epidemics nearly half of the adult male population in Finland have demonstrable IgG antibodies against chlamydia TWAR, and this is a typical situation in other developed countries as well. In the present study sera were collected in 1983 to 1985. A TWAR epidemic started in Finland in 1985, and the prevalence of low titer (≧ 32) antibodies against this chlamydia in 65 % and only a few elevated IgG or IgA antibodies in controls fits well to an antibody status during interepidemic period. However, patients with chronic coronary heart disease and acute myocardial infarction had higher prevalence than expected and significantly higher antibody titers against TWAR chlamydia than the computer matched controls.

Primary TWAR infections are characterized by a predominant IgM response, delayed IgG response and a weak or absent IgA response, whereas secondary infections are characterized with absence of IgM response, and prompt IgG and IgA responses. The patients in the present study had high, stable IgG and IgA titers, and no IgM antibodies in micro-IF test. Altogether, the antibody findings in patients with CCHD and AMI suggest a chronic TWAR chlamydia infection in them. The abnormal responses in Re-LPS EIA further point to an involment of chlamydia in coronary heart disease. While not opposing the established risk factors, this unexpected association presents an alternative basis for prophylaxis, treatment and diagnosis of coronary heart disease.

Demonstration of circulating chlamydial LPS immunocomplexes and LPS in patients with myocardial infarction

Patients and controls

The AMI patient group consisted of 44 consecutive young, middleaged and old men and women admitted to Helsinki University Central Hospital in 1985 and 1986 because of acute myocardial infarction. The inclusion criteria were: resident in Helsinki or immediate neighbourhood, onset of symptoms within 36 hours prior to the admittance, and verification of acute myocardial infarction by typical changes in ECG and elevation of serum creatinine phosphokinase MB-isoenzyme activity.

Controls were 44 healthy men and women recruited as a computer based random sample from official inhabitant records of the city of Helsinki from the same areas as patients. None of the controls had any signs or symptoms of coronary heart disease as judged by negative history and a normal resting ECG. The controls were selected to represent the same age range as the patients and they were invited to the examination sequantially one at the time. Serum samples for immunocomplex determinations were taken on admission and 4 weeks later.

The CCHD patient group consisted of 104 young, middle aged and old men and women from Helsinki area whose CCHD was diagnosed by angiography in 1985 and 1986.

Detection of LPS-immunocomplexes

A. 96-well EIA-plates were coated with mouse monoclonal antibody to chlamydial LPS. This monoclonal antibody reacts with the KDO-trisaccharide unit specific to chlamydial LPS and not reacting with Re-LPS or Acinetobacter LPS. Serum samples diluted 1:100 and 1:1000 with PBS containing 10 % fetal calf serum were added into the wells and incubated for 2 hrs at 37 °C. After washing alkaline-phosphatase conjugated anti-human -IgM or -IgG diluted with PBS-fetal calf serum was added and incubated for 2 hours at 37 °C. After washing again the substrate, 1 mg of paranitrophenylphosphate in 1 ml of diethanolamine buffer, pH 0.6, was added into the wells and optical density (O.D.) values were measured at 405 nm wavelength after incubation for 30 min. at +37 °C. The amount of chlamydia specific immunocomplexes was expressed at EIA titers: highest serum dilution giving O.D. value of 0.2.

B. 96-well EIA plates were coated with mouse monoclonal antibody against human IgM, ( μ-chain specific). Serum samples were diluted as in method A were added to the wells. After incubation and washings alkaline-phosphatase conjugated monoclonal antibody to chlamydial LPS was added and EIA procedure was continued as in method A.

Results

The majority of patients had demonstrable chlamydia LPS - containing immunocomplexes in their sera. The results are shown in Table 2.

Table 2

| Presence in sera of immunocomplexes reactive with chlamydial LPS specific monoclonal antibody | | | | | |
|---|---|---|---|---|---|
| Ig-class | AMI Patients | p-value | Controls | p-value | CCHD |
| IgM-complexes | 24/44 (55 %) | <0.0005 | 6/44 (14 %) | 0.025 | 35/104 (34 %) |
| IgG-complexes | 4/44 (5 %) | n.s. | 1/44 (3 %) | 0.0025 | 26/104 (25 %) |
| Total complexes | 26/44 (59 %) | <0.0001 | 7/44 (16 %) | 0.0001 | 53/104 (51 %) |

Detection of chlamydial LPS in patient sera.

Sera were tested for the presence of chlamydia LPS by using commercial chlamydial antigen detection kit for chlamydial LPS (IDEIA™, Boots-Celltech Diagnostics Ltd., Slough, UK), diluted 1 to 8 in dilution buffer included in the kit. LPS was detected in 5/19 (26 %) of AMI patient sera and in none of 19 control sera studied.

The demonstration of chlamydial LPS immune complexes or LPS in AMI patients confirm the association of chlamydial infections with AMI. In addition, the presence of immune complexes further suggest that chlamydial LPS might play important role in the pathogenesis and development of AMI and CCHD.

**Claims**

1. A method for screening the risks of heart infarction, for the diagnosis of myocardial infarction and coronary heart disease, and for the monitoring of treatment of patients with acute myocardial infarction or coronary heart disease, wherein a sample of a patient is tested for the presence of chlamydia or chlamydial antibodies.

2. The method according to claim 1, wherein the sample is tested for the presence of chlamydial saccharides or immune complexes containing chlamydial components.

3. The method according to claim 1 or 2, wherein the disease is cerebral infarction or vascular embolization.

4. A method according to any one of claims 1 to 3, wherein the chlamydia is Chlamydia TWAR.

5. Use of a drug effective against chlamydia for the manufacture of a pharmaceutical preparation to be used in the treatment or prophylaxis of coronary heart disease.

6. The use according to claim 5, wherein said chlamydia is Chlamydia TWAR.

**Patentansprüche**

1. Verfahren zur Überprüfung der Risiken eines Herzinfarkts, zur Diagnose des Myokardinfarkts und von Krankheiten der Herzkrangefäße sowie zur Überwachung der Behandlung von Patienten mit akutem Myokardinfarkt oder akuter Erkrankung der Herzkranzgefäße, bei dem eine Probe eines Patienten auf die Anwesenheit von Chlamydien oder Chlamydia-Antikörpern geprüft wird.

2. Verfahren nach Anspruch 1, bei dem die Probe auf die Anwesenheit von Chlamydia-Sacchariden oder Immunkomplexen, die Chlamydia-Komponenten enthalten, geprüft wird.

3. Verfahren nach Anspruch 1 oder 2, bei dem die Krankheit ein Hirninfarkt oder eine Gefäßverstopfung ist.

EP 0 377 722 B1

**4.** Verfahren nach einem der Ansprüche 1 bis 3, bei dem die Chlamydien *Chlamydia* TWAR sind.

**5.** Verwendung einer gegen Chlamydien wirksamen Droge zur Herstellung einer pharmazeutischen Zubereitung zur Behandlung oder Prophylaxe von Erkrankungen der Herzkranzgefäße.

**6.** Verwendung nach Anspruch 5, bei dem die Chlamydien *Chlamydia* TWAR sind.

**Revendications**

**1.** Procédé pour le dépistage des risques d'un infarctus cardiaque, pour le diagnostic d'un infarctus du myocarde et d'une maladie coronarienne, et pour le suivi du traitement des patients qui ont un infarctus aigu du myocarde ou une maladie coronarienne, selon lequel on recherche dans un prélèvement effectué sur un patient la présence de chlamydia ou d'anticorps de chlamydia.

**2.** Procédé selon la revendication 1, selon lequel on recherche dans le prélèvement la présence de saccharides de chlamydia ou d'immun-complexes renfermant des composants de chlamydia.

**3.** Procédé selon l'une des revendications 1 ou 2, selon lequel la maladie est l'infarctus cérébral ou l'embolisation vasculaire.

**4.** Procédé selon l'une des revendications 1 à 3, selon lequel la chlamydia est la chlamydia TWAR.

**5.** Utilisation d'un produit pharmaceutique efficace contre la chlamydia pour la fabrication d'une préparation pharmaceutique destinée au traitement ou à la prophylaxie d'une maladie coronarienne.

**6.** Utilisation selon la revendication 5, selon laquelle la chlamydia est la chlamydia TWAR.

10